# EUROPEAN PATENT APPLICATION

(11) **EP 3 942 991 A1**
(43) Date of publication of application: **26.01.2022**
(21) Application number: 20773220.7
(22) Date of filing: 06.03.2020
(51) Int. Cl.: A61B 1/00, A61B 1/045

(54) **ENDOSCOPE PROCESSOR DEVICE, MEDICAL IMAGE PROCESSING DEVICE, OPERATION METHOD THEREOF, AND PROGRAM FOR MEDICAL IMAGE PROCESSING DEVICE**

(30) Priority: 20.03.2019 JP 2019053545
(71) Applicant: FUJIFILM Corporation, Tokyo 106-8620 (JP)
(72) Inventor: TATSUTA, Takeichi, Ashigarakami-gun, Kanagawa 258-8538 (JP); FUKUDA, Takeshi, Ashigarakami-gun, Kanagawa 258-8538 (JP)
(74) Representative: Jeffrey, Philip Michael
(86) International application number: PCT/JP2020/009646
(87) International publication number: WO 2020/189334

(57) **Abstract**

There are provided a processor device for an endoscope, a medical image processing device, a method of operating the medical image processing device, and a program for the medical image processing device that can use picked-up images of a subject subjected to length measurement by an endoscope as teacher data for machine learning.

A signal processing unit (39) of a processor device for an endoscope includes an observation distance detection section (52) and a learning section (56). A first picked-up image and a second picked-up image, which are subjected to signal processing by the signal processing unit (39), are stored by a static image storage control unit (43). The first picked-up image is a picked-up image that is acquired from the image pickup of a subject irradiated with auxiliary measurement light, and the second picked-up image is a picked-up image that is acquired from the image pickup of the subject not irradiated with the auxiliary measurement light.

## Description

### BACKGROUND OF THE PRESENT INVENTION

### 1. Field of the invention

The present invention relates to a processor device for an endoscope, a medical image processing device, a method of operating the medical image processing device, and a program for the medical image processing device.

### 2. Description of the Related Art

A distance to an object to be observed, the size of the object to be observed, and the like are acquired in the field of an endoscope. In, for example, WO2018/051680A, a subject is irradiated with auxiliary measurement light from an auxiliary light irradiation unit provided in an endoscope and a spot is formed on the subject. Accordingly, a processor device for an endoscope specifies the position of the spot from a picked-up image that is obtained from the image pickup of the subject. Then, the processor device for an endoscope detects an observation distance from the position of the spot. Further, the processor device for an endoscope sets a measurement marker, which shows the actual size of an object to be observed included in the subject, according to the observation distance, and displays the set measurement marker in the picked-up image. The size of the object to be observed can be measured using the measurement marker displayed in the picked-up image.

On the other hand, after the names of a region of interest, such as the names of organs, the names of diseases, and symptoms, and the images of the regions of interest are learned using machine learning, such as deep learning, in the field of image diagnosis using medical images, a region of interest is detected and the name of the region of interest are determined from the medical images. In order to detect the region of interest and determine the name of the region of interest with high accuracy, learning using a large amount of good-quality teacher data corresponding to the purpose is absolutely necessary in the machine learning.

### SUMMARY OF THE INVENTION

Even in the field of an endoscope, it is examined that the picked-up image obtained from the image pickup of the subject is used as teacher data for machine learning. Particularly, since the subject, which is subjected to length measurement by the processor device for an endoscope disclosed in WO2018/051680A, is likely to include the region of interest, and is also be a subject as an object for machine learning, the picked-up image of the subject is required to be used as the teacher data for machine learning.

However, since the spot irradiated with the auxiliary measurement light is captured on the subject, the picked-up image cannot be used as the teacher data for machine learning in the processor device for an endoscope disclosed in WO2018/051680A. A normal endoscopic image without the spot irradiated with the auxiliary measurement light is required as the teacher data for machine learning.

An object of the present invention is to provide a processor device for an endoscope, a medical image processing device, a method of operating the medical image processing device, and a program for the medical image processing device that can use picked-up images of a subject subjected to length measurement by an endoscope as teacher data for machine learning.

A processor device for an endoscope according to an aspect of the present invention comprises a processor. The processor acquires picked-up images that are picked up by an endoscope; and acquires at least one of a first picked-up image, which is acquired from image pickup of a subject irradiated with auxiliary measurement light, or second picked-up images, which are acquired before and after irradiation of the auxiliary measurement light and are acquired from image pickup of the subject not irradiated with the auxiliary measurement light, among the picked-up images of the endoscope as a teacher data candidate for machine learning.

It is preferable that the processor acquires the first picked-up image or the second picked-up images in a multi-frame mode where the auxiliary measurement light is alternately turned on and dimmed according to a preset light emission pattern, the first picked-up image is a picked-up image acquired in a case where the auxiliary measurement light is turned on, and the second picked-up image is a picked-up image acquired in a case where the auxiliary measurement light is dimmed.

It is preferable that the processor acquires the first picked-up image or the second picked-up images in a multi-frame mode where the auxiliary measurement light is alternately turned on and dimmed according to a preset light emission pattern, the first picked-up image is a picked-up image acquired in a case where the auxiliary measurement light is turned on and a freeze switch is operated, and the second picked-up image is a picked-up image acquired in a case where the auxiliary measurement light is dimmed and the freeze switch is operated.

It is preferable that the processor acquires the first picked-up image or the second picked-up images in an always-on mode where the auxiliary measurement light is always turned on, the first picked-up image is an image acquired in a state where the subject is irradiated with the auxiliary measurement light in a case where a freeze switch is operated or an image which is acquired in a state where the subject is irradiated with the auxiliary measurement light before and after an operation of the freeze switch and of which a shape of an object to be observed is similar to that of an image acquired in a case where the freeze switch is operated, and the second picked-up image is a picked-up image acquired before a mode is switched to the always-on mode or after the always-on mode is switched to the other mode.

It is preferable that the processor acquires a lesion area-candidate image, in which a range including a specific region formed on the subject by the auxiliary measurement light is cut out from the first picked-up image or the second picked-up image, as the teacher data candidate.

It is preferable that the processor specifies a position of a specific region formed on the subject by the auxiliary measurement light and acquires position information of the specific region together with the first picked-up image or the second picked-up images.

It is preferable that the processor detects an observation distance from the specified position of the specific region and acquires the observation distance together with the first picked-up image or the second picked-up images.

It is preferable that the processor acquires diagnostic information from a diagnostic information management device that manages diagnostic information about the first picked-up image or the second picked-up images and acquires the diagnostic information together with the first picked-up image or the second picked-up images.

A medical image processing device according to another aspect of the present invention comprises a processor. The processor acquires picked-up images picked up by an endoscope and performs image processing on a medical image; acquires at least one of a first picked-up image, which is acquired from image pickup of a subject irradiated with auxiliary measurement light, or second picked-up images, which are acquired before and after irradiation of the auxiliary measurement light and are acquired from image pickup of the subject not irradiated with the auxiliary measurement light, among the picked-up images of the endoscope as a teacher data candidate for machine learning; recognizes a lesion area from the medical image; acquires diagnostic information from a diagnostic information management device that manages diagnostic information about the first picked-up image or the second picked-up images; and learns to generate a discriminant model, which is used to recognize the lesion area, using the teacher data candidate and the diagnostic information.

A method of operating a medical image processing device according to another aspect of the present invention acquires picked-up images picked up by an endoscope and performs image processing on a medical image. The method comprises: a step of acquiring at least one of a first picked-up image, which is acquired from image pickup of a subject irradiated with auxiliary measurement light, or second picked-up images, which are acquired before and after irradiation of the auxiliary measurement light and are acquired from image pickup of the subject not irradiated with the auxiliary measurement light, among the picked-up images of the endoscope as a teacher data candidate for machine learning; a step of recognizing a lesion area from the medical image; a step of acquiring diagnostic information from a diagnostic information management device that manages diagnostic information about the first picked-up image or the second picked-up images; and a step of learning to generate a discriminant model, which is used to recognize the lesion area, using the teacher data candidate and the diagnostic information.

A program for a medical image processing device according to another aspect of the present invention is installed in a medical image processing device that acquires picked-up images picked up by an endoscope and performs image processing on a medical image. The program causes a computer to realize: a function to acquire at least one of a first picked-up image, which is acquired from image pickup of a subject irradiated with auxiliary measurement light, or second picked-up images, which are acquired before and after irradiation of the auxiliary measurement light and are acquired from image pickup of the subject not irradiated with the auxiliary measurement light, among the picked-up images of the endoscope as a teacher data candidate for machine learning; a function to recognize a lesion area from the medical image; a function to acquire diagnostic information from a diagnostic information management device that manages diagnostic information about the first picked-up image or the second picked-up images; and a function to learn to generate a discriminant model, which is used to recognize the lesion area, using the teacher data candidate and the diagnostic information.

According to the present invention, picked-up images of a subject subjected to length measurement by an endoscope can be used as teacher data for machine learning.

### BRIEF DESCRIPTION OF THE DRAWINGS

Fig. 1 is a diagram showing the appearance of an endoscope system.
Fig. 2 is a plan view of a distal end part of an endoscope.
Fig. 3 is a block diagram showing the functions of the endoscope system.
Fig. 4 is a block diagram of an auxiliary measurement light-emitting unit.
Fig. 5 is a diagram illustrating a relationship between the distal end part of the endoscope and a near end PN, an intermediate vicinity PM, and a far end PF in a range R1 of an observation distance.
Fig. 6 is a block diagram showing the functions of a signal processing unit.
Fig. 7 is a diagram illustrating the outline of the configuration of a multi-frame image.
Fig. 8 is a diagram illustrating light source control, image pickup control, and storage control in a case where a static image-acquisition instruction is given in a length measurement mode.
(A) and (B) of Fig. 9 are diagrams illustrating a method of storing a first picked-up image and a second picked-up image.
Fig. 10 is a diagram illustrating an example in which the first picked-up image is displayed.
Fig. 11 is a diagram illustrating light source control, image pickup control, and storage control in a case where a static image-acquisition instruction is given in a second embodiment.
Fig. 12 is a diagram illustrating light source control, image pickup control, and storage control in a case where a static image-acquisition instruction is given in a modification example of the second embodiment.
(A), (B), (C), and (D) of Fig. 13 are diagrams illustrating a method of acquiring lesion area-candidate images, in which ranges including specific regions are cut out in a first embodiment and the modification example of the second embodiment, as a teacher data candidate.

### DESCRIPTION OF THE PREFERRED EMBODIMENTS

### [First embodiment]

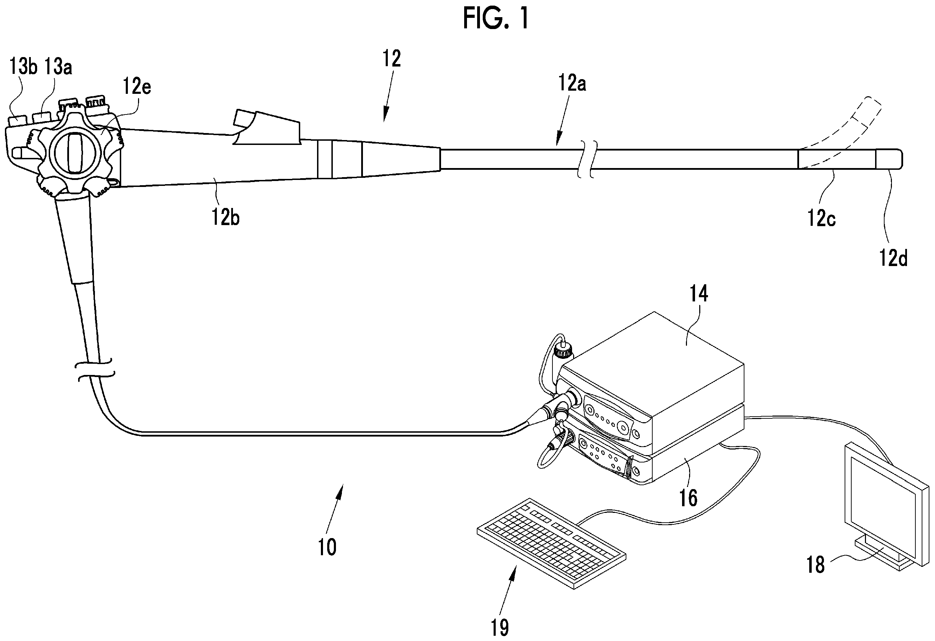

As shown in Fig. 1, an endoscope system 10 includes an endoscope 12, a light source device 14, a processor device 16, a monitor 18, and a user interface 19. The endoscope 12 is optically connected to the light source device 14, and is electrically connected to the processor device 16. The processor device 16 is electrically connected to the monitor 18 (display unit) that displays an image. The user interface 19 is connected to the processor device 16, and is used for various setting operations and the like for the processor device 16. The user interface 19 includes a mouse and the like in addition to a keyboard shown in Fig. 1.

An application program for causing each device to function as a component of the endoscope system 10 is installed in each device. Further, the application program may be installed from a recording medium, such as a CD-ROM, or may be installed after being downloaded from a storage device of a server connected through a network, such as the internet.

The endoscope 12 includes an insertion part 12a that is to be inserted into an object to be examined, an operation part 12b that is provided at the proximal end portion of the insertion part 12a, and a bendable part 12c and a distal end part 12d that are provided on the distal end side of the insertion part 12a. The bendable part 12c operates to be bent by the operation of angle knobs 12e of the operation part 12b. The distal end part 12d is made to face in a desired direction by the bending operation of the bendable part 12c.

The endoscope 12 has a normal mode and a length measurement mode, and these two modes are switched by a mode changeover switch 13a that is provided on the operation part 12b of the endoscope 12. The normal mode is a mode where an object to be observed is illuminated with illumination light. In the length measurement mode, an object to be observed is illuminated with illumination light or auxiliary measurement light and a measurement marker to be used to measure the size and the like of the object to be observed is displayed in a picked-up image obtained from the image pickup of the object to be observed. The auxiliary measurement light is light that is used to measure a subject.

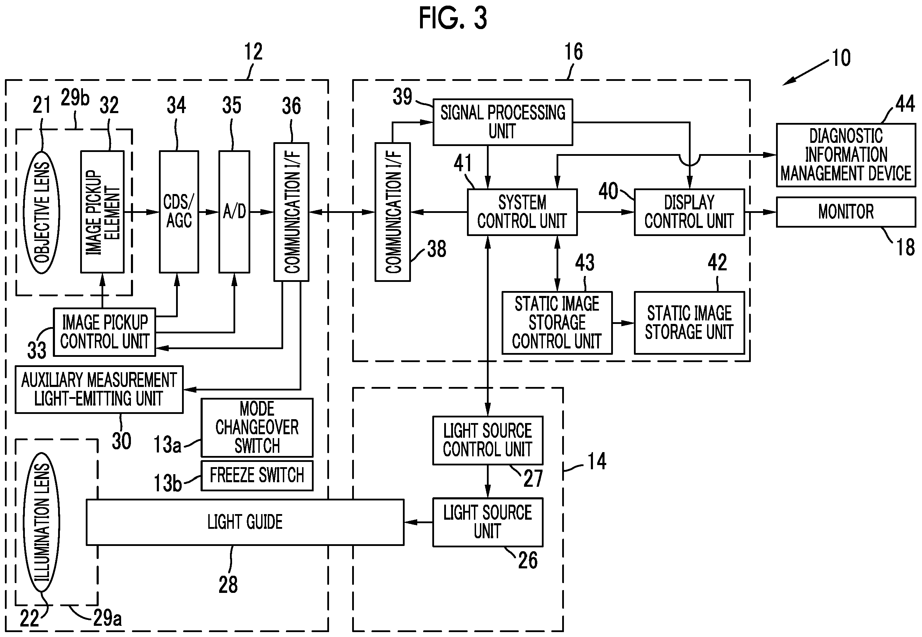

Further, the operation part 12b of the endoscope 12 is provided with a freeze switch 13b (static image-acquisition instruction unit) that is used to give a static image-acquisition instruction to acquire the static image of a picked-up image. In a case where a user operates the freeze switch 13b, the screen of the monitor 18 is frozen and displayed and an alert sound (for example, "beep") informing the acquisition of a static image is generated together. Then, the static images of the picked-up image, which are obtained before and after the operation timing of the freeze switch 13b, are stored in a static image storage unit 42 (see Fig. 3) provided in the processor device 16.

The static image storage unit 42 is a storage unit, such as a hard disk or a universal serial bus (USB) memory. In a case where the processor device 16 can be connected to a network, the static image of the picked-up image may be stored in a static image storage server (not shown), which is connected to a network, instead of or in addition to the static image storage unit 42.

A static image-acquisition instruction may be given using an operation device other than the freeze switch 13b. For example, a foot pedal may be connected to the processor device 16, and a static image-acquisition instruction may be given in a case where a user operates the foot pedal (not shown) with a foot. A foot pedal may also be used to switch a mode. Further, a static image-acquisition instruction may be given or a mode may be switched by the input of voice, the input of a sight line, the input of a gesture, or the like.

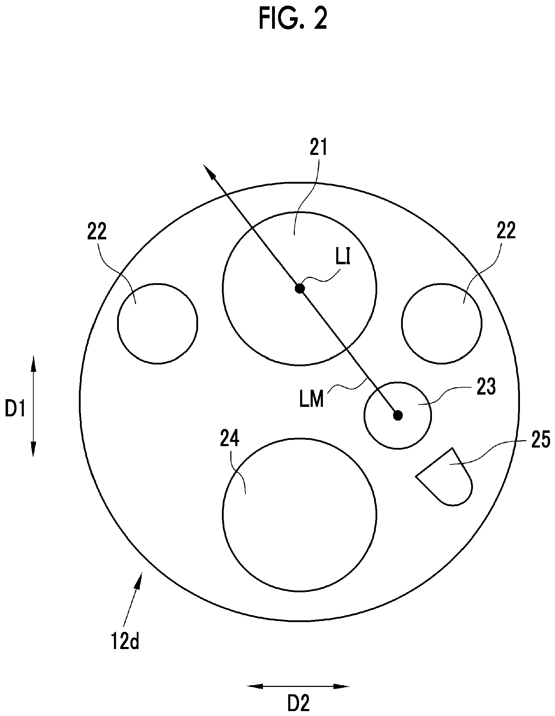

As shown in Fig. 2, the distal end part of the endoscope 12 has a substantially circular shape; and is provided with an objective lens 21 that is positioned closest to a subject among optical members of an image pickup optical system of the endoscope 12, two illumination lenses 22 that are used to irradiate a subject with the illumination light, an auxiliary measurement lens 23 that is used to illuminate a subject with the auxiliary measurement light to be described later, an opening 24 that allows a treatment tool to protrude toward a subject, and an air/water supply nozzle 25 that is used to supply air and water.

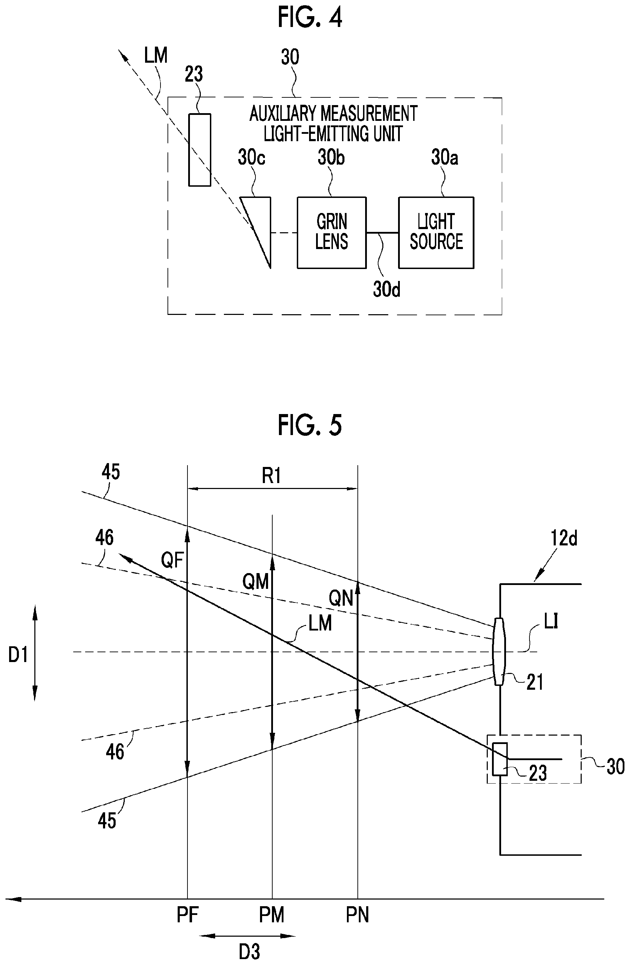

An optical axis LI (see Fig. 5) of the objective lens 21 extends in a direction perpendicular to the plane of paper. A vertical first direction D1 is orthogonal to the optical axis LI, and a horizontal second direction D2 is orthogonal to the optical axis LI and the first direction D1. The objective lens 21 and the auxiliary measurement lens 23 are arranged in the first direction D1.

As shown in Fig. 3, the light source device 14 comprises a light source unit 26 and a light source control unit 27. The light source unit 26 (illumination light source unit) generates the illumination light that is used to illuminate a subject. The illumination light emitted from the light source unit 26 is incident on a light guide 28, and a subject is irradiated with the illumination light through the illumination lenses 22. It is preferable that a white light source emitting white light, a plurality of light sources, which include a white light source and a light source emitting another color light (for example, a blue light source emitting blue light), or the like is used as a light source of the illumination light in the light source unit 26. The light source control unit 27 is connected to a system control unit 41 of the processor device 16. The light source control unit 27 controls the light source unit 26 on the basis of an instruction given from the system control unit 41. The system control unit 41 (light emitting control unit) not only gives an instruction related to light source control to the light source control unit 27 but also controls a light source 30a (see Fig. 4) of an auxiliary measurement light-emitting unit 30. The details of the light source control performed by the system control unit 41 will be described later.

The distal end part 12d of the endoscope 12 is provided with an illumination optical system 29a, an image pickup optical system 29b, and an auxiliary measurement light-emitting unit 30. The illumination optical system 29a includes the illumination lenses 22, and an object to be observed is irradiated with light, which is emitted from the light guide 28, through the illumination lenses 22. The image pickup optical system 29b includes the objective lens 21 and an image pickup element 32. Light reflected from the object to be observed is incident on the image pickup element 32 through the objective lens 21. Accordingly, the reflected image of the object to be observed is formed on the image pickup element 32.

The image pickup element 32 is a color image pickup sensor, and picks up the reflected image of an object to be examined and outputs image signals. It is preferable that the image pickup element 32 is a charge coupled device (CCD) image pickup sensor, a complementary metal-oxide semiconductor (CMOS) image pickup sensor, or the like. The image pickup element 32 used in an embodiment of the present invention is a color image pickup sensor that is used to obtain RGB image signals corresponding to three colors of R (red), G (green), and B (blue). The image pickup element 32 is controlled by an image pickup control unit 33. A complementary color image pickup element, which is provided with color filters corresponding to complementary colors, that is, C (cyan), M (magenta), Y (yellow), and G (green), may be used as the image pickup element 32.

Image signals output from the image pickup element 32 are transmitted to a CDS/AGC circuit 34. The CDS/AGC circuit 34 performs correlated double sampling (CDS) or auto gain control (AGC) on the image signals that are analog signals. The image signals, which have been transmitted through the CDS/AGC circuit 34, are converted into digital image signals by an analog/digital converter (A/D converter) 35. The digital image signals, which have been subjected to A/D conversion, are input to the processor device 16 through a communication interface (I/F) 36.

The processor device 16 comprises a communication interface (I/F) 38 that is connected to the communication I/F 36 of the endoscope 12, a signal processing unit 39, a display control unit 40, and the system control unit 41. The communication I/F receives the image signals, which are transmitted from the communication I/F 36 of the endoscope 12, and transmits the image signals to the signal processing unit 39. A memory, which temporarily stores the image signals received from the communication I/F 38, is built in the signal processing unit 39, and the signal processing unit 39 processes an image signal group, which is a set of the image signals stored in the memory, to generate the picked-up image.

The processor device 16 has a function as a medical image processing device, and the signal processing unit 39 acquires the picked-up image of the endoscope and acquires diagnostic information about the picked-up image to perform machine learning as described later. In a case where the endoscope 12 is set to the length measurement mode, the signal processing unit 39 may be adapted to perform structure-emphasis processing of emphasizing structures, such as blood vessels, or color difference-emphasis processing of increasing a color difference between a normal area and a lesion area of the object to be observed on the picked-up image.

The display control unit 40 causes the monitor 18 to display the picked-up image that is generated by the signal processing unit 39. The system control unit 41 performs the control of the image pickup element 32 through the image pickup control unit 33 provided in the endoscope 12. The image pickup control unit 33 also performs the control of the CDS/AGC 34 and the A/D 35 together with the control of the image pickup element 32. A static image storage control unit 43 performs control related to the static image of the picked-up image to be stored in the static image storage unit 42. The static image storage control unit 43 performs control to be described later in a case where a static image-acquisition instruction is given once in the length measurement mode.

As shown in Fig. 4, the auxiliary measurement light-emitting unit 30 (auxiliary measurement light source unit) comprises a light source 30a, a GradientIndex (GRIN) lens 30b, a prism 30c, an optical fiber 30d, and the auxiliary measurement lens 23. The light source 30a is to emit light having a color that can be detected by pixels of the image pickup element 32 (specifically visible light), and includes a light-emitting element, such as a laser light source LD (laser diode) or a light emitting diode (LED), and a condenser lens that condenses light emitted from the light-emitting element.

The light emitted from the light source 30a is, for example, red light having a wavelength of preferably 600 nm or more and 750 nm or less, more preferably 600 nm or more and 700 nm or less, and most preferably 630 nm or more and 660 nm or less. Alternatively, green light having a wavelength of 495 nm or more and 570 nm or less may be used. The light source 30a is controlled by the system control unit 41 and emits light on the basis of an instruction given from the system control unit 41. The optical fiber 30d guides the light emitted from the light source 30a to the GRIN lens 30b. In order to convert the light, which is emitted from the light source 30a, into the auxiliary measurement light used to measure a subject, the GRIN lens 30b converts the light, which is guided from the light source 30a by the optical fiber 30d, into highly coherent light again.

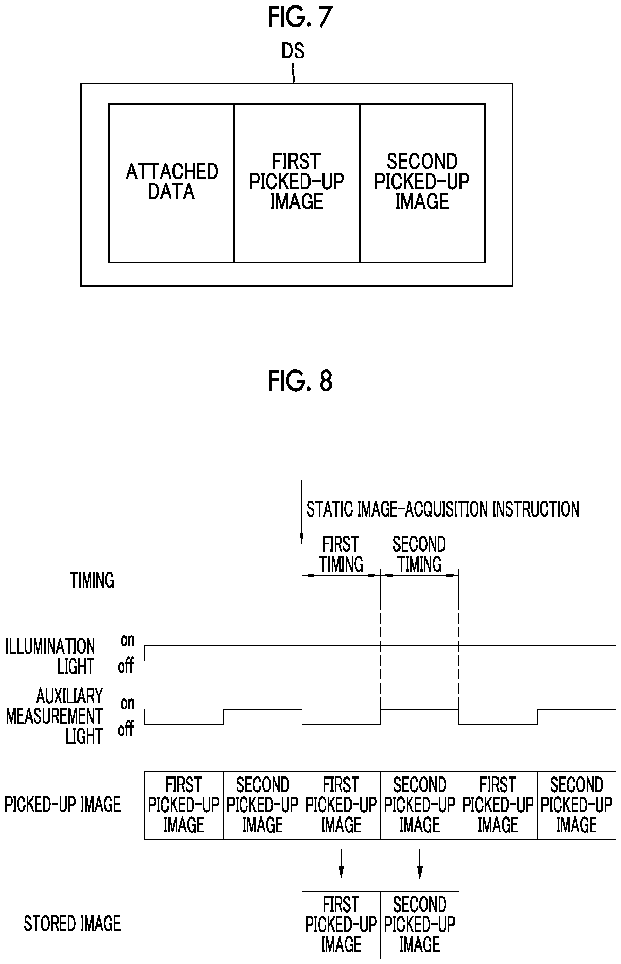

The prism 30c is an optical member that is used to change the travel direction of the auxiliary measurement light converted by the GRIN lens 30b. The prism 30c changes the travel direction of the auxiliary measurement light so that the auxiliary measurement light crosses the visual field of the image pickup optical system including the objective lens 21 and lens groups. The details of the travel direction of the auxiliary measurement light will also be described later. A subject is irradiated with the auxiliary measurement light, which is emitted from the prism 30c, through the auxiliary measurement lens 23. In a case where the subject is irradiated with the auxiliary measurement light, a spot SP as a circular region (specific region) is formed on the subject (see (B) of Fig. 7). The position of the spot SP is specified by a position specifying section 51, and a measurement marker showing an actual size is set according to the position of the spot SP. The set measurement marker is superimposed and displayed on the picked-up image.

An auxiliary measurement slit formed in the distal end part 12d of the endoscope may be used instead of the auxiliary measurement lens 23. Further, it is preferable that an anti-reflection coating (AR coating) (anti-reflection portion) is provided on the auxiliary measurement lens 23. The reason why the anti-reflection coating is provided as described above is that it is difficult for the position specifying section 51 to be described later to recognize the position of the spot SP formed on the subject by the auxiliary measurement light in a case where the auxiliary measurement light is reflected without being transmitted through the auxiliary measurement lens 23 and a ratio of the auxiliary measurement light with which a subject is irradiated is reduced.

The auxiliary measurement light-emitting unit 30 has only to be capable of emitting the auxiliary measurement light to the visual field of the image pickup optical system. For example, the light source 30a may be provided in the light source device and light emitted from the light source 30a may be guided to the GRIN lens 30b by the optical fiber 30d. Further, the prism 30c may not be used, a diffractive optical element (DOE) may be used instead of the GRIN lens 30b, and the directions of the light source 30a, the DOE, and an optical fiber, which guides light emitted from the light source 30a to the DOE, may be inclined with respect to the optical axis LI so that the auxiliary measurement light is emitted in a direction crossing the visual field of the image pickup optical system.

With regard to the travel direction of the auxiliary measurement light, the auxiliary measurement light is emitted in a state where an optical axis LM of the auxiliary measurement light crosses the optical axis LI of the objective lens 21 as shown in Fig. 5. In a case where a subject can be observed in a range R1 of an observation distance, it is understood that the positions (points where the respective arrows QN, QM, and QF cross the optical axis LM) of the spot SP, which is formed on the subject by the auxiliary measurement light, in image pickup ranges (shown by arrows QN, QM, and QF) at a near end PN, an intermediate vicinity PM, and a far end PF of the range R1 are different from each other. The image pickup angle of view of the image pickup optical system is represented by a region between two solid lines 45, and measurement is performed in a central region (a region between two dotted lines 46), in which an aberration is small, of this image pickup angle of view.

Since the auxiliary measurement light is emitted in a state where the optical axis LM of the auxiliary measurement light crosses the optical axis LI as described above, sensitivity to the movement of the position of the spot with respect to a change in the observation distance is high. Accordingly, the size of the subject can be measured with high accuracy. Then, the image of the subject illuminated with the auxiliary measurement light is picked up by the image pickup element 32, so that a picked-up image including the spot SP is obtained. In the picked-up image, the position of the spot SP varies depending on a relationship between the optical axis LI of the objective lens 21 and the optical axis LM of the auxiliary measurement light and an observation distance. However, the number of pixels showing the same actual size (for example, 5 mm) is increased in the case of a short observation distance, and the number of pixels showing the same actual size (for example, 5 mm) is reduced in the case of a long observation distance.

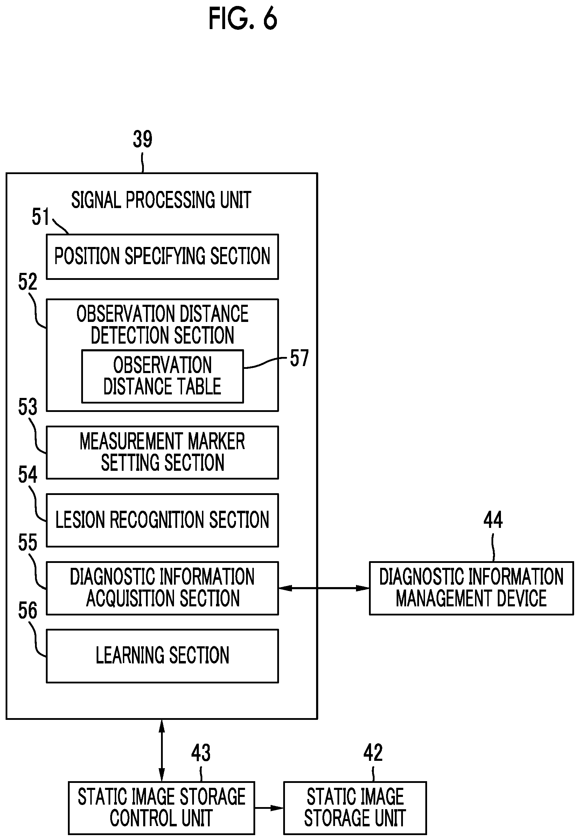

As shown in Fig. 6, the signal processing unit 39 of the processor device 16 comprises a position specifying section 51, an observation distance detection section 52, a measurement marker setting section 53, a lesion recognition section 54, a diagnostic information acquisition section 55, and a learning section 56.

A first picked-up image and a second picked-up image, which are subjected to signal processing by the signal processing unit 39, are stored by the static image storage control unit 43 as described later. The first picked-up image is a picked-up image that is acquired from the image pickup of a subject irradiated with the auxiliary measurement light in the length measurement mode, and the second picked-up image is a picked-up image that is acquired from the image pickup of the subject not irradiated with the auxiliary measurement light in the length measurement mode.

In this embodiment, the first picked-up image and the second picked-up image are data called a multi-frame image and are stored in the static image storage unit 42. This is a medical image data in which image data of a plurality of frames form one data set DS as shown in Fig. 7, and is used for the storage of a video or the like. The data set DS includes image data of the first picked-up image and the second picked-up image and attached data attached to the image data.

The static image storage control unit 43 corresponds to a teacher data candidate-acquisition unit of the claims. That is, the static image storage control unit 43 functions to store the first picked-up image and the second picked-up image in the static image storage unit 42 as medical images used for diagnosis and the like and to acquire the second picked-up image as a teacher data candidate for machine learning, and stores information, which shows that, for example, the second picked-up image is a teacher data candidate, in the static image storage unit 42 as the attached data of the data set DS. Alternatively, the static image storage control unit 43 may store the second picked-up image, which is acquired as a teacher data candidate, in a storage device (not shown) or the like that is included in a diagnostic information management device 44 separately from the static image storage unit 42.

The position specifying section 51 specifies the position of the spot SP on the basis of the first picked-up image. Specifically, the position specifying section 51 specifies coordinate information about the position of the spot SP. The spot SP is displayed as a substantially circular red region that includes a large amount of components corresponding to the color of the auxiliary measurement light in the first picked-up image. Accordingly, the position specifying section 51 specifies the position of the spot SP from the substantially circular red region. As a method of specifying a position, for example, a picked-up image is binarized and the centroid of a white portion (pixel at which the signal intensity is higher than a binarized threshold value) of the binarized image is specified as the position of the spot SP.

The observation distance detection section 52 detects an observation distance on the basis of the position of the spot SP. The observation distance detection section 52 detects an observation distance from the position of the spot SP with reference to an observation distance table 57 in which a relationship between the position of the spot SP in the first picked-up image and an observation distance is stored. The measurement marker setting section 53 sets a measurement marker that has an actual size and corresponds to the observation distance detected by the observation distance detection section 52.

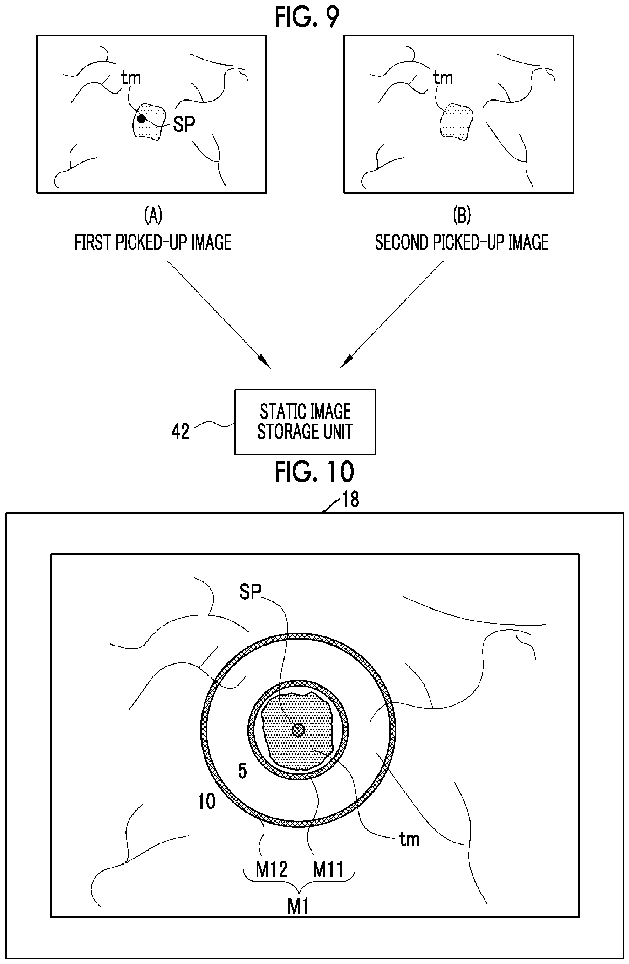

The measurement marker setting section 53 creates the data of the measurement marker, which corresponds to the observation distance detected by the observation distance detection section 52, in the first picked-up image. This measurement marker is a circular measurement marker M1 (see Fig. 10) representing an actual size of, for example, "5 mm" and "10 mm" in a case where this measurement marker is superimposed and displayed on the first picked-up image.

It is preferable that coordinate information about the position of the spot SP specified by the position specifying section 51, the observation distance detected by the observation distance detection section 52, or the measurement marker created by the measurement marker setting section 53 is stored as the attached data of the data set DS.

The lesion recognition section 54 analyzes the second picked-up image that is acquired by the signal processing unit 39, and performs recognition processing. Detection processing of detecting a region of interest from the second picked-up image is performed as the recognition processing that is performed by the lesion recognition section 54. In this embodiment, the lesion recognition section 54 performs recognition processing of detecting a lesion area, which is a region of interest, from the second picked-up image. In this case, the lesion recognition section 54 divides the second picked-up image into a plurality of small regions, for example, square regions of which the number is equal to the numbers of pixels. After that, image feature quantities are calculated from the divided second picked-up image. Subsequently, it is determined whether or not each small region is a lesion area on the basis of the calculated feature quantities. It is preferable that a machine learning algorithm, such as artificial intelligence (AI), a convolutional neural network, or a deep learning, is used as this determination method.

As the recognition processing that is performed by the lesion recognition section 54, discrimination processing of discriminating the type and the like of a lesion may be performed for the lesion area recognized from the second picked-up image or discrimination processing may be performed for the entire second picked-up image to acquire a discrimination result.

The diagnostic information acquisition section 55 acquires diagnostic information about the first picked-up image or the second picked-up image from the diagnostic information management device 44. The medical record of a patient as an object to be examined is acquired as the diagnostic information acquired from the diagnostic information management device 44. The medical record is information in which the progress and the like of medical care or examination for a patient are recorded, and includes, for example, a record, such as the name, the gender and age, the name of a disease, major symptoms, the contents of prescription or treatment, or the medical history of a patient. Information about the lesion area that is subjected to the recognition processing by the lesion recognition section 54 and diagnostic information about the first picked-up image or the second picked-up image that is acquired by the diagnostic information acquisition section 55 are stored as the attached data of the data set DS.

In a case where the second picked-up image as a teacher data candidate is acquired by the static image storage control unit 43, the learning section 56 performs machine learning using a plurality of second picked-up images and a plurality of pieces of the information about the lesion area and the diagnostic information that are stored as attached data. Specifically, the learning section 56 generates a discriminant model using a plurality of second picked-up images and a plurality of pieces of the information about the lesion area, which is subjected to the recognition processing, and the diagnostic information by a machine learning method, such as deep learning. Since the second picked-up image is a picked-up image in which a region of interest as an object to be observed is included and is a normal endoscopic image that is not irradiated with the auxiliary measurement light, the second picked-up image can be used as the teacher data of machine learning. Further, since the information about the lesion area, the diagnostic information, and the like are also attached as the attached data, a user does not need to input the information about the lesion area, the diagnostic information, and the like in a case where machine learning is performed. The generated discriminant model is used for the recognition processing for the lesion area that is performed by the lesion recognition section 54. Accordingly, the accuracy of the recognition processing performed by the lesion recognition section 54 is improved as the second picked-up images as a teacher data candidate are accumulated.

Light source control and image pickup control performed by the system control unit 41 and static image storage control performed by the static image storage control unit 43 will be described. In a case where the endoscope 12 is set to the normal mode, the system control unit 41 instructs the light source unit 26 of the light source device 14 to always emit the illumination light. Accordingly, the illumination light is emitted from the light source unit 26. A subject is irradiated with the illumination light through the light guide 28. The light source 30a of the auxiliary measurement light-emitting unit 30 stops in the case of the normal mode. Further, the static image storage control unit 43 stores the second picked-up image, which is obtained from the image pickup of a subject illuminated with the illumination light, in the static image storage unit 42 in a case where a static image-acquisition instruction is given once in the normal mode.

On the other hand, in a case where the endoscope 12 is set to the length measurement mode, the system control unit 41 controls the illumination light of the light source unit 26 of the light source device 14 and the auxiliary measurement light of the light source 30a of the auxiliary measurement light-emitting unit 30 according to a preset light emission pattern. Specifically, in a case where the freeze switch 13b is operated in a multi-frame mode where the illumination light is always turned on and the auxiliary measurement light is alternately turned on and dimmed as shown in Fig. 8, the first picked-up image and the second picked-up image are acquired.

In a case where the freeze switch 13b is operated to give a static image-acquisition instruction in the length measurement mode, a first picked-up image is picked up at a first timing including the static image-acquisition instruction in a state where the illumination light is turned on (on) and the auxiliary measurement light is turned on (on) as shown in Fig. 8. A second picked-up image is picked up at a second timing when the first timing has passed in a state where the illumination light is turned on (on) and the auxiliary measurement light is dimmed (off) so that the intensity of the auxiliary measurement light is equal to or lower than a certain value.

Since the static image-acquisition instruction is included in the first timing in the example shown in Fig. 8, the first picked-up image and the second picked-up image are picked up in this order. However, in a case where the static image-acquisition instruction is included in the second timing, the first picked-up image may be picked up after the second picked-up image is picked up.

Then, the static image storage control unit 43 stores a multi-frame image, which includes the static image of the first picked-up image and the static image of the second picked-up image, as a stored image that is to be stored in the static image storage unit 42. The multi-frame image is formed of one first picked-up image and one second picked-up image that are picked up at the time of the static image-acquisition instruction in the example shown in Fig. 7, but is not limited thereto. A plurality of first picked-up images or a plurality of second picked-up images picked up before and after the static image-acquisition instruction may be included in one multi-frame image as in a case where a video is acquired.

Further, it is preferable to display the first picked-up image and the second picked-up image on the monitor 18 to notify that a static image is recorded for a certain period of time after a static image-acquisition instruction is given. After the first picked-up image and the second picked-up image are stored, the length measurement mode is maintained as it is but may be automatically switched to the normal mode.

As shown in (A) of Fig. 9, the first picked-up image is an image in which the spot SP is captured. On the other hand, as shown in (B) of Fig. 9, the second picked-up image is an normal endoscopic image and is used for the morphological observation or diagnosis of a tumor tm on the basis of a color, a shape, a range, and the like.

In a case where the display control unit 40 causes the monitor 18 to display the first picked-up image, the first picked-up image is not limited to an example shown in (A) of Fig. 9 and a measurement marker M1 may be superimposed and displayed on the first picked-up image as shown in Fig. 10.

The measurement marker M1 is a concentric circular measurement marker that is formed of two concentric circles having different sizes on the basis of the position of one spot SP, and "5" and "10" attached to the lower left portions of two concentric circles M11 and M12 forming the measurement marker M1 represent actual sizes of "5 mm" and "10 mm". In this case, the measurement marker M1 is superimposed and displayed on the tumor tm in post-processing after endoscopic diagnosis, such as after a procedure, so that the first picked-up image is used in a case where a user want to measure a size and a distance in detail. The post-processing may be performed by a personal computer (PC) or the like other than the endoscope system 10.

Further, in a case where the display control unit 40 causes the monitor 18 to display the first picked-up image, the display control unit 40 may cause the monitor 18 to display the results of the recognition processing performed by the lesion recognition section 54. For example, as shown in Fig. 10, only the tumor tm that is a lesion area recognized by the lesion recognition section 54 may be highlighted by a change in color or the type (not shown) of a recognized lesion may be displayed. Furthermore, it is preferable that the first picked-up image on which the measurement marker M1 is superimposed and displayed remains in a medical record as an evidence image in a case where a tumor is to be extirpated and it is determined that the tumor needs to be discarded (a tumor or the like in a case where the tumor is larger or smaller than a predetermined standard).

As described above, the first picked-up image in which the spot SP is captured and the second picked-up image, which is a normal endoscopic image, can be acquired in a case where a static image-acquisition instruction is given once. Accordingly, the size and the like of an object to be observed can be measured using the first picked-up image, and the second picked-up image can be used as the teacher data of machine learning.

The static image storage control unit 43 stores a multi-frame image including the first picked-up image and the second picked-up image, but is not limited thereto. The static image storage control unit 43 may store each of the first picked-up image and the second picked-up image as a single image file. In a case where a single image file formed of, for example, Exif, DICOM format, or the like as the data format of the picked-up image is employed, it is preferable that coordinate information about the position of the spot SP is the metadata of the first picked-up image and the second picked-up image. Further, in a case where coordinate information about the position of the spot SP and information and diagnostic information about a lesion area are used as separate files, such as ini files, the coordinate information about the position of the spot SP and the information and diagnostic information about a lesion area are stored in association with the first picked-up image and the second picked-up image.

### [Second embodiment]

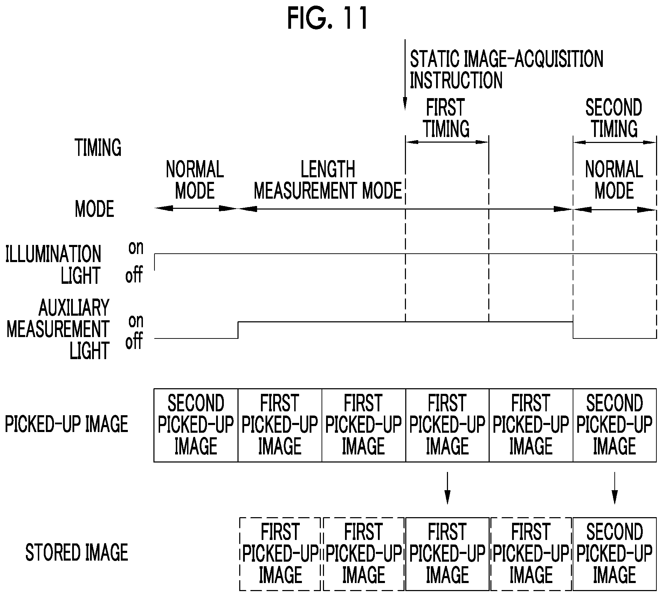

In the first embodiment, the first picked-up image and the second picked-up image are acquired in the multi-frame mode where the auxiliary measurement light is alternately turned on and dimmed. However, in a second embodiment, the first picked-up image is acquired in an always-on mode and the second picked-up image is acquired in the normal mode. As shown in Fig. 11, in the case of the always-on mode, the system control unit 41 always turns on the auxiliary measurement light. Since configuration other than the light source control and image pickup control performed by the system control unit 41 and the static image storage control performed by the static image storage control unit 43 is the same as that of the first embodiment, the description thereof will be omitted.

In a case where the endoscope 12 is set to the normal mode, the system control unit 41 instructs the light source unit 26 of the light source device 14 to always emit illumination light as in the first embodiment. Then, in a case where a mode is switched to the length measurement mode from the normal mode by the operation of the mode changeover switch 13a, the system control unit 41 picks up the first picked-up image in a case where the freeze switch 13b is operated in the always-on mode where the illumination light and the auxiliary measurement light are always turned on.

In a case where the freeze switch 13b is operated to give a static image-acquisition instruction in the length measurement mode, the first picked-up image is picked up at a first timing including the static image-acquisition instruction in a state where the illumination light is turned on (on) and the auxiliary measurement light is turned on (on) as shown in Fig. 11.

On the other hand, the second picked-up image is acquired before a mode is switched to the length measurement mode or after the length measurement mode is switched to the other mode. In the example shown in Fig. 11, the second picked-up image is picked up at a second timing when the length measurement mode has been switched to the normal mode in a state where the illumination light is turned on (on) and the auxiliary measurement light is dimmed (off) so that the intensity of the auxiliary measurement light is equal to or lower than a certain value. A flow until the static image storage control unit 43 stores the first picked-up image and the second picked-up image after acquiring the first picked-up image and the second picked-up image is the same as that of the first embodiment. In this embodiment, it is preferable that a mode is automatically switched to the normal mode from the length measurement mode after the first picked-up image is picked up. Accordingly, the second timing can be obtained in a short time from the first timing when the first picked-up image is picked up, and the second picked-up image can be picked up.

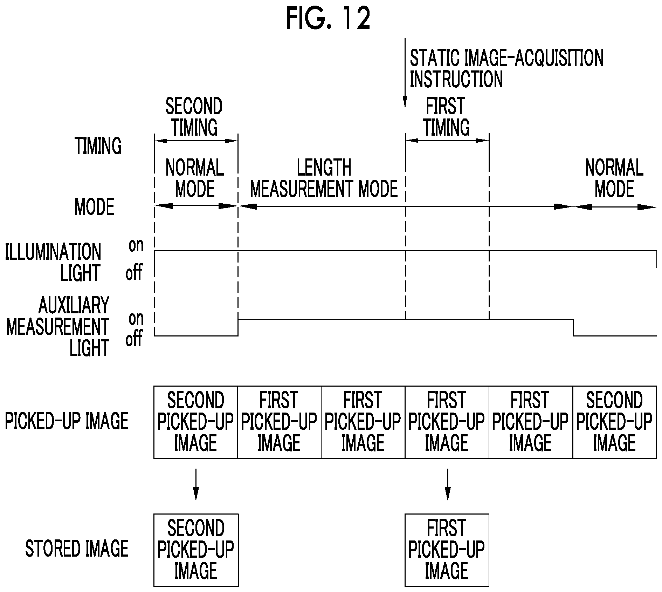

In the example shown in Fig. 11, the second picked-up image is picked up at the second timing when the length measurement mode has been switched to the normal mode. However, as shown in Fig. 12, the second picked-up image, which is picked up at the second timing in the normal mode that is not yet switched to the length measurement mode, is temporarily stored in a memory or the like. Then, in a case where the freeze switch 13b is operated to give a static image-acquisition instruction after a mode is switched to the length measurement mode, the temporarily stored second picked-up image may be acquired together with the first picked-up image that is picked up. Alternatively, both a second picked-up image obtained before a mode is switched to the length measurement mode and a second picked-up image obtained after the length measurement mode is switched to the normal mode may be acquired.

As described above, the first picked-up image in which the spot SP is captured and the second picked-up image, which is a normal endoscopic image, can be acquired in a case where a static image-acquisition instruction is given once. Accordingly, the second embodiment has the same effect as the first embodiment.

In the second embodiment, the static image storage control unit 43 acquires only the first picked-up image, which is picked up at the first timing including the static image-acquisition instruction, in the length measurement mode. However, the present invention is not limited thereto, and picked-up images which are acquired in a state where a subject is irradiated with the auxiliary measurement light before and after a static image-acquisition instruction is given and of which the shape of an object to be observed is similar to that of an image picked up at the first timing, may be acquired as the first picked-up image.

Specifically, first picked-up images (first picked-up images surrounded by broken lines among the stored images shown in Fig. 11) other than the first picked-up image picked up at the first timing (a first picked-up image surrounded by a solid line among the stored images shown in Fig. 11) in the length measurement mode correspond to the above-mentioned picked-up images. All these first picked-up images acquired in the length measurement mode may be acquired, or the feature quantity of a first picked-up image picked up at the first timing and the feature quantities of first picked-up images other than the first picked-up image picked up at the first timing may be calculated and a first picked-up image of which the shape of an object to be observed is most similar to that of the first picked-up image picked up at the first timing may be acquired. Further, in a case where the second picked-up image is acquired after the length measurement mode is switched to the normal mode, it is preferable that first picked-up images close to the second timing even among the first picked-up images, that is, first picked-up images picked up after the first timing are acquired. Furthermore, in a case where the second picked-up image is acquired before a mode is switched to the length measurement mode, it is preferable that first picked-up images picked up before the first timing are acquired.

An example where all of the lesion recognition section 54, the teacher data candidate-acquisition unit (static image storage control unit 43), the diagnostic information acquisition section 55, and the learning section 56 are provided in the endoscope system 10 has been described in each of the embodiments. However, any one of them may be provided in the endoscope system and the others of them may be provided in an external medical image processing device connected to the endoscope system. For example, only the lesion recognition section is provided in the endoscope system, and the teacher data candidate-acquisition unit, the diagnostic information acquisition section, and the learning section are provided in the external medical image processing device. Then, the learning section of the external medical image processing device causes the lesion recognition section of the endoscope system to learn.

In each of the embodiments, in a case where the second picked-up image is picked up, the auxiliary measurement light is dimmed so that the intensity of the auxiliary measurement light is equal to or lower than a certain value. However, the auxiliary measurement light may be turned off without being dimmed. Further, in each of the embodiments, the teacher data candidate-acquisition unit (static image storage control unit 43) acquires the first picked-up image in which the spot SP is captured and the second picked-up image, which is a normal endoscopic image and acquires the second picked-up image as a teacher data candidate. However, the teacher data candidate-acquisition unit (static image storage control unit 43) is not limited thereto, and may acquire at least one of the first picked-up image or the second picked-up image as a teacher data candidate. In a case where the first picked-up image is acquired as a teacher data candidate, for example, portions other than a portion in which the spot SP is captured may be used for the machine learning performed by the learning section.

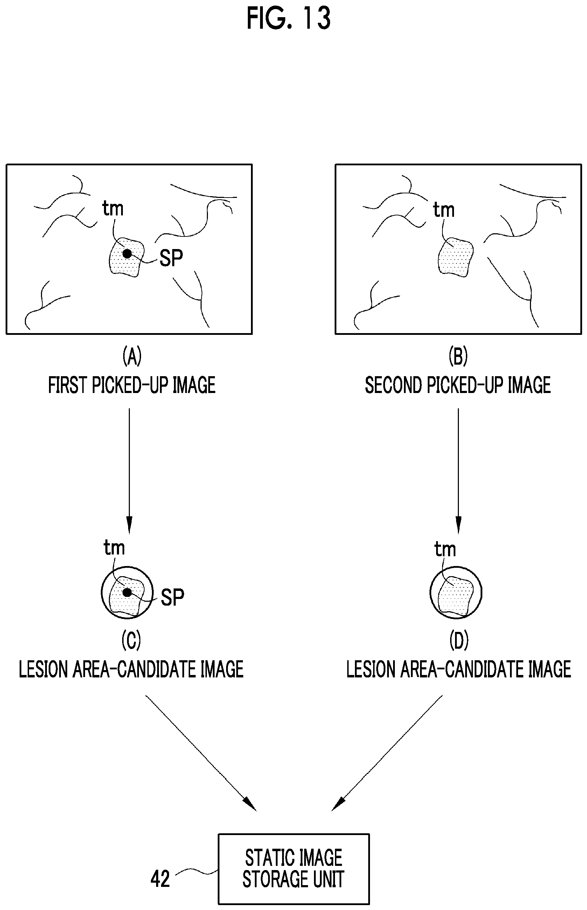

Furthermore, not the entire image of the above-mentioned first or second picked-up image but a lesion area-candidate image in which the periphery of the spot SP is cut out may be acquired as the teacher data candidate that is acquired by the teacher data candidate-acquisition unit (static image storage control unit 43). In this case, in a case where the freeze switch 13b is operated to give a static image-acquisition instruction, as shown in (A), (B), (C), and (D) of Fig. 13, the teacher data candidate-acquisition unit (static image storage control unit 43) creates a lesion area-candidate image (see (C) and (D) of Fig. 13) in which the periphery of the spot SP is cut out from the first picked-up image or the second picked-up image (see (A) and (B) of Fig. 13) using the position information of the spot SP.

A circular range, which has a center at the position of the spot SP and has a diameter corresponding to a predetermined number of pixels, is cut out as the periphery of the spot SP in the example shown in (A), (B), (C), and (D) of Fig. 13. However, the periphery of the spot SP is not limited thereto and may be a range that includes the position of the spot SP and is formed in a predetermined shape. Alternatively, only the range of a tumor tm, which is subjected to the recognition processing by the lesion recognition section 54, may be cut out. Then, the lesion area-candidate image, which is cut out, is stored in the static image storage unit 42 or the like. A portion in which the spot SP is captured, that is, a portion which a medical doctor as a user wants to observe may be naturally an object to be learned. Accordingly, the learning section 56 performs machine learning as in each of the embodiments using the lesion area-candidate image in which the periphery of the spot SP is cut out. Therefore, since machine learning can be performed on the basis of the lesion area-candidate image that can be selected by a medical doctor, the accuracy of the recognition processing performed by the lesion recognition section 54 is further improved. The teacher data candidate acquired by the teacher data candidate-acquisition unit may be any one of a lesion area-candidate image in which the periphery of the spot SP is cut out from the first picked-up image or a lesion area-candidate image in which the periphery of the spot SP is cut out from the second picked-up image.

In each of the embodiments, the hardware structures of processing units, which perform various kinds of processing, such as the signal processing unit 39, the display control unit 40, and the system control unit 41, are various processors to be described later. Various processors include: a central processing unit (CPU) that is a general-purpose processor functioning as various processing units by executing software (program); a graphical processing unit (GPU); a programmable logic device (PLD) that is a processor of which the circuit configuration can be changed after manufacture, such as a field programmable gate array (FPGA); a dedicated electrical circuit that is a processor having circuit configuration designed exclusively to perform various kinds of processing; and the like.

One processing unit may be formed of one of these various processors, or may be formed of a combination of two or more same kind or different kinds of processors (for example, a plurality of FPGAs, a combination of a CPU and an FPGA, a combination of a CPU and a GPU, or the like). Further, a plurality of processing units may be formed of one processor. As an example where a plurality of processing units are formed of one processor, first, there is an aspect where one processor is formed of a combination of one or more CPUs and software as typified by a computer, such as a client or a server, and functions as a plurality of processing units. Second, there is an aspect where a processor fulfilling the functions of the entire system, which includes a plurality of processing units, by one integrated circuit (IC) chip as typified by System On Chip (SoC) or the like is used. In this way, various processing units are formed using one or more of the above-mentioned various processors as hardware structures.

In addition, the hardware structures of these various processors are more specifically electrical circuitry where circuit elements, such as semiconductor elements, are combined. Explanation of References

- 10:: endoscope system
- 12:: endoscope
- 12a:: insertion part
- 12b:: operation part
- 12c:: bendable part
- 12d:: distal end part
- 12e:: angle knob
- 13a:: mode changeover switch
- 13b:: freeze switch
- 14:: light source device
- 16:: processor device
- 18:: monitor
- 19:: user interface
- 21:: objective lens
- 22:: illumination lens
- 23:: auxiliary measurement lens
- 24:: opening
- 25:: air/water supply nozzle
- 26:: light source unit
- 27:: light source control unit
- 28:: light guide
- 29a:: illumination optical system
- 29b:: image pickup optical system
- 30:: auxiliary measurement light-emitting unit
- 30a:: light source
- 30b:: GRIN lens
- 30c:: prism
- 30d:: optical fiber
- 32:: image pickup element
- 33:: image pickup control unit
- 34:: CDS/AGC circuit
- 35:: A/D
- 36:: communication interface (I/F)
- 38:: communication interface (I/F)
- 39:: signal processing unit
- 40:: display control unit
- 41:: system control unit
- 42:: static image storage unit
- 43:: static image storage control unit
- 44:: diagnostic information management device
- 45:: solid line
- 46:: dotted line
- 51:: position specifying section
- 52:: observation distance detection section
- 53:: measurement marker setting section
- 54:: lesion recognition section
- 55:: diagnostic information acquisition section
- 56:: learning section
- 57:: observation distance table
- D1:: first direction
- D2:: second direction
- DS:: data set
- LI:: optical axis
- LM:: optical axis
- M1:: measurement marker
- M11:: concentric circle
- M12:: concentric circle
- PF:: far end
- PM:: intermediate vicinity
- PN:: near end
- QF:: arrow
- QM:: arrow
- QN:: arrow
- R1:: range
- SP:: spot
- tm:: tumor

## Claims

1. A processor device for an endoscope comprising:
a processor,
wherein the processor acquires picked-up images that are picked up by an endoscope, and acquires at least one of a first picked-up image, which is acquired from image pickup of a subject irradiated with auxiliary measurement light, or second picked-up images, which are acquired before and after irradiation of the auxiliary measurement light and are acquired from image pickup of the subject not irradiated with the auxiliary measurement light, among the picked-up images of the endoscope as a teacher data candidate for machine learning.

2. The processor device for an endoscope according to claim 1,
wherein the processor acquires the first picked-up image or the second picked-up images in a multi-frame mode where the auxiliary measurement light is alternately turned on and dimmed according to a preset light emission pattern,
the first picked-up image is a picked-up image that is acquired in a case where the auxiliary measurement light is turned on, and
the second picked-up image is a picked-up image that is acquired in a case where the auxiliary measurement light is dimmed.

3. The processor device for an endoscope according to claim 1,
wherein the processor acquires the first picked-up image or the second picked-up images in a multi-frame mode where the auxiliary measurement light is alternately turned on and dimmed according to a preset light emission pattern,
the first picked-up image is a picked-up image that is acquired in a case where the auxiliary measurement light is turned on and a freeze switch is operated, and
the second picked-up image is a picked-up image that is acquired in a case where the auxiliary measurement light is dimmed and the freeze switch is operated.

4. The processor device for an endoscope according to claim 1,
wherein the processor acquires the first picked-up image or the second picked-up images in an always-on mode where the auxiliary measurement light is always turned on,
the first picked-up image is an image that is acquired in a state where the subject is irradiated with the auxiliary measurement light in a case where a freeze switch is operated or an image which is acquired in a state where the subject is irradiated with the auxiliary measurement light before and after an operation of the freeze switch and of which a shape of an object to be observed is similar to that of an image acquired in a case where the freeze switch is operated, and
the second picked-up image is a picked-up image that is acquired before a mode is switched to the always-on mode or after the always-on mode is switched to the other mode.

5. The processor device for an endoscope according to claim 3 or 4,
wherein the processor acquires a lesion area-candidate image, in which a range including a specific region formed on the subject by the auxiliary measurement light is cut out from the first picked-up image or the second picked-up image, as the teacher data candidate.

6. The processor device for an endoscope according to any one of claims 1 to 5,
wherein the processor specifies a position of a specific region formed on the subject by the auxiliary measurement light, and acquires position information of the specific region together with the first picked-up image or the second picked-up images.

7. The processor device for an endoscope according to claim 6,
wherein the processor detects an observation distance from the specified position of the specific region, and acquires the observation distance together with the first picked-up image or the second picked-up images.

8. The processor device for an endoscope according to any one of claims 1 to 5,
wherein the processor acquires diagnostic information from a diagnostic information management device that manages diagnostic information about the first picked-up image or the second picked-up images, and acquires the diagnostic information together with the first picked-up image or the second picked-up images.

9. A medical image processing device comprising:
a processor,
wherein the processor acquires picked-up images picked up by an endoscope and performs image processing on a medical image, acquires at least one of a first picked-up image, which is acquired from image pickup of a subject irradiated with auxiliary measurement light, or second picked-up images, which are acquired before and after irradiation of the auxiliary measurement light and are acquired from image pickup of the subject not irradiated with the auxiliary measurement light, among the picked-up images of the endoscope as a teacher data candidate for machine learning, recognizes a lesion area from the medical image, acquires diagnostic information from a diagnostic information management device that manages diagnostic information about the first picked-up image or the second picked-up images, and learns to generate a discriminant model, which is used to recognize the lesion area, using the teacher data candidate and the diagnostic information.

10. A method of operating a medical image processing device that acquires picked-up images picked up by an endoscope and performs image processing on a medical image, the method comprising:
a step of acquiring at least one of a first picked-up image, which is acquired from image pickup of a subject irradiated with auxiliary measurement light, or second picked-up images, which are acquired before and after irradiation of the auxiliary measurement light and are acquired from image pickup of the subject not irradiated with the auxiliary measurement light, among the picked-up images of the endoscope as a teacher data candidate for machine learning;
a step of recognizing a lesion area from the medical image;
a step of acquiring diagnostic information from a diagnostic information management device that manages diagnostic information about the first picked-up image or the second picked-up images; and
a step of learning to generate a discriminant model, which is used to recognize the lesion area, using the teacher data candidate and the diagnostic information.

11. A program for a medical image processing device that is installed in a medical image processing device acquiring picked-up images picked up by an endoscope and performing image processing on a medical image, the program causing a computer to realize:
a function to acquire at least one of a first picked-up image, which is acquired from image pickup of a subject irradiated with auxiliary measurement light, or second picked-up images, which are acquired before and after irradiation of the auxiliary measurement light and are acquired from image pickup of the subject not irradiated with the auxiliary measurement light, among the picked-up images of the endoscope as a teacher data candidate for machine learning;
a function to recognize a lesion area from the medical image;
a function to acquire diagnostic information from a diagnostic information management device that manages diagnostic information about the first picked-up image or the second picked-up images; and
a function to learn to generate a discriminant model, which is used to recognize the lesion area, using the teacher data candidate and the diagnostic information.
